# EUROPEAN PATENT APPLICATION

(11) **EP 4 791 035 A1**
(43) Date of publication of application: **12.08.2026**
(21) Application number: 25159279.6
(22) Date of filing: 21.02.2025
(51) Int. Cl.: H04R 1/10, G10K 11/178, H04R 5/033

(54) **HEARING PROTECTION AND COMMUNICATION SYSTEM WITH ADAPTABLE NOISE REDUCTION**

(30) Priority: 07.02.2025 EP 25156466; 07.02.2025 EP 25156472; 07.02.2025 EP 25156479
(71) Applicant: INVISIO A/S, 2650 Hvidovre (DK)
(72) Inventor: DAHL, Jonas Burup, 2650 Hvidovre (DK); PEDERSEN, Sine Storm, 2650 Hvidovre (DK); ANDERSEN, Mathias Bach, 2650 Hvidovre (DK); BØNNELYKKE, Jesper Rye, 2650 Hvidovre (DK); PETERSEN, Jacob, 2650 Hvidovre (DK); GRÜNEWALD, Manuel, 2650 Hvidovre (DK); BODEN, Richard, 2650 Hvidovre (DK)
(74) Representative: Guardian IP Consulting I/S

(57) **Abstract**

A communication system for communication in harsh environments includes a hearing protection headset with left and right earcups. Each earcup comprises a speaker unit, a cavity with an opening adapted for the user's ear, and a flexible seal for acoustic sealing. The earcups provide passive sound attenuation, reducing external noise. The headset features an adaptor element with a central void and a rim that, when inserted between the earcup and flexible seal, increases the cavity volume for enhanced sound attenuation. The headset also includes an active noise reduction (ANR) circuit that generates anti-noise signals to cancel out external noise. The ANR circuit operates in two modes, depending on whether the adaptor element is inserted or not.

## Description

### TECHNICAL FIELD

The present disclosure relates to a hearing protection and communication system with adaptable noise reduction, particularly to be used in a harsh and demanding environment.

### BACKGROUND

Communication and hearing protection systems are essential for professionals operating in noisy and mission critical environments to work safely and effectively while protecting their hearing. Clear and undistorted communication is vital for military and public safety professionals operating in harsh and demanding environments. Clear and undistorted communication is essential for soldiers, police, rescue personnel, fire fighters, and other task forces as it facilitates improved coordination among team members and enhances safety and security by reducing misunderstandings. Rapid and swift communication facilitates quick decision-making and responses to changing situations. Besides communication capabilities, it is outermost important to shield individuals who requires to operate in harsh environments against exposure to harmful external noise levels such as vehicle noise, gun shots, explosions, etc.

Hearing protection devices are generally known and used amount soldiers, police forces etc. for noise attenuation. Typically, passive hearing protecting devices such as foam earplugs or earmuffs may be used to physically blocking or dampening sound waves thereby protecting the user against harmful high noise exposure; however this type of hearing protecting devices blocks out all type of sound, which can be extremely problematic for many types of operations, where the user is required to maintain both situational awareness of the surroundings and clear communication with other team members via radio frequency (RF) communication connection.

The communication equipment utilized by military and public safety professionals across different agencies, units and teams may span widely dependent on the environment the professionals are in. For example, a military person may operate armored vehicles such as tanks, light armor, and amphibious assault vehicles during combat situations in harsh and demanding environments. Such a person called a combat vehicle crewman (CVC) and are exposed to extremely high noise levels inside the armored vehicles, which may pose significant risks to their hearing, overall health and ability to operate the vehicle. Accordingly, when the CVC is inside the vehicle, and operates the vehicle, it is of uttermost importance that the high noise levels can be reduced. However, when the CVC operating the light armor, may be outside the vehicle, and on foot, for example in a forest, it is uttermost importance that not all external sounds are reduced - and that the CVC is provided with situational awareness.

Thus, there is a need to develop a hearing protection and communication system to be worn by a user in harsh environments configured to adapt to different situations.

### SUMMARY

It is an object of the present disclosure to provide a communication system for communicating in a harsh environment that is configured to adapt to different situations.

It is further an object of the present disclosure to provide a communication system for communicating in a harsh environment that is configured to adapt to a first situation with low noise and to a second situation with high noise.

Accord to a first aspect, the present disclosure provides a communication system for communicating in a harsh environment, comprising:
- a hearing protection headset to be worn by a user in the harsh environment, said headset comprising:
- a left and a right earcup, wherein each of said earcup comprises:
   ∘ a speaker unit;
   ∘ a cavity with a opening adapted to accommodate the ear of the user;
   ∘ a flexible seal around a periphery of the opening to form an ear opening, wherein the flexible seal is configured to acoustically seal the cavity when worn by the user;
   wherein each of said earcup and flexible seal defines a first cavity volume, wherein the cavity first cavity volume contributes to provide passive sound attenuation, whereby each earcup is configured to attenuate external sounds from the harsh environment.

Further, said headset comprises:
- an adaptor element having a central void and a rim having a thickness such that the central void defines a void volume, and wherein the adaptor element is configured to be removably arranged between the opening and the flexible seal such that the rim rest against the periphery of the opening, thereby effectively increasing the first cavity volume to a second cavity volume defined by the first cavity volume and the void volume, thereby increasing the passive sound attenuation.

It is advantageous to have a headset according to the first aspect configured to be physically altered by the addition of an adapter element, such that the affective volume of each of the earcups may be changed. This enables the headset to me modified to be worn by a user operating in different types of harsh and demanding environments, like inside a tracked armored vehicle or on the ground in open air. When a user is operating in a mounted configuration such as inside a armored vehicle, like a tank or caterpillars, the notice level can be extreme, and the user may need extensive hearing protecting to be able to operate under such conditions. By increasing the earcup cavity volume of the headset, the level of passive sound attenuation will consequently increase as well along the larger formfactor of the headset (e.g. The headset may extend further away from the head of the user). When a user is operating in a dismounted configuration, such as under open-air, the noise level may be lower as compared to the mounted configuration. Even though a user in a dismounted configuration may benefit from the additional passive hearing protection provided by the increased earcup cavity volume, the larger formfactor is undesirable as the headset should fit over the head of the user in combination with a ballistic helmet and be agile in narrow spaces etc. Additionally, the slight increase in weight the adapter adaptor elements would add to the headset in undesirable for the dismounted user. Thus, having a headset capable of altering the earcup cavity volume is highly advantageous for users operating in harsh and demanding environment including both dismounted and mounded configurations. It is advantageous to have at least one speaker in each of said earcups, such that audio signals may be played to the user, thereby enabling the headset to be both a communication- and hearing protection headset for providing clear and undistorted communication in harsh and demanding environments.

In a preferred embodiment according to the first aspect, said headset further comprise an active noise reduction (ANR) circuit, configured to attenuate the external sounds from the harsh environment by generating anti-noise signals, via said speaker units, that destructively interfere with the external sounds, wherein the ANR circuit is configured to operate in two ANR modes:
- a first ANR mode, or
- a second ANR mode.

It is advantageous to combine passive hearing protection with active noise reduction (ANR) or active noise cancellation (ANC), to achieve a higher degree of external sound attenuation, compared to the passive blocking of sound alone. Having a headset configured to operate in two different ANR modes enables the headset to be used in two different modes of operation requiring different levels or way of performing ANR, due to power requirements, characteristics of external noise, or physical change of the earcup cavity volume (e.g. A first cavity volume or a second cavity volume). Thus, the communication system according to the first aspect may be used by personal operating in harsh and demanding environments requiring at least a first ANR mode or a second ANR mode to provide the specific level of anti-noise signal generation for a specific type of environment, e.g. User is vehicles, urban terrain, jungle, or dessert, as each individual harsh environment may expose the user to a different external noise environment (e.g. Different dominant external noise frequency bands)

In a preferred embodiment according to the first aspect, the first ANR mode is applied when the adaptor element is not removably placed between the opening and the flexible seal and the second ANR mode is applied when the adaptor element is removably placed between the opening and the flexible seal.

It is advantageous that the communication system according to the disclosure is configured to apply the first ANR mode when the adapter element is not attached to the earcups respectively and apply the second ANR mode when the adapter element is attached to the earcups respectively, such that the first ANR mode may be tuned and optimized for the generating anti-noise signals when the earcups have a first cavity volume and the second ANR mode may be tuned and optimized for generating generating anti-noise signals, then earcups have a second cavity volume. It is particularly advantageous that the headset may recognize that the adapter elements are attached to the earcups and in response apply the second ANR mode.

In a most preferred embodiment, the communication system according to the present disclosure is configured such that when in the first ANR mode, the ANR circuit is configured to operate at a first level of noise reduction that is dependent on the first cavity volume, preferably such that the first level of noise reduction is matched to the first cavity volume and its corresponding passive sound attenuation, and when in the second ANR mode, the ANR circuit is configured to operate at a second level of noise reduction that is dependent on the second cavity volume, preferably such that the second level of noise reduction is matched to the second cavity volume and its corresponding passive sound attenuation.

Having a headset configured to operate in two different ANR modes enables the headset to be used in two different physicals configurations, where the earcup cavity volume is varied. By applying a first ANR mode when the hearing protection headset is having a first cavity volume and a first ANR mode when the hearing protection headset is having second cavity volume enables the headset to optimal active noise reduction (ANR) in both physical configurations, such that the hearing protection headset may be used by a user in either a dismounted configuration or a mounted configuration.

In a most preferred embodiment, the communication system as described in the most preferred embodiment is configured such that the first level of noise reduction is lower than the second level of noise reduction, such that when in the first ANR mode, external sounds are reduced less by the ANR circuit than when in the second ANR mode.

In another preferred embodiment, the communication system as described in the most preferred embodiment is configured such that the first level of noise reduction is related to a first frequency range and the second level of noise reduction is related to a second frequency range, such that when in the first ANR mode, external sounds are reduced more in the first frequency range by the ANR circuit than when in the second ANR mode and when in the second ANR mode, external sounds are reduced more in the second frequency range by the ANR circuit than when in the first ANR mode.

This is advantageous, when the first ANR mode may applied by the hearing protection headset, when the user is operating in a dismounted configuration, where the external sounds are dominant in a first frequency range, such as a pink noise frequency profile, and then the second ANR mode is applied by the hearing protection headset, when he user is operating in a mounted configuration, where the external sounds are dominant in a second frequency range, such as in the low frequency spectrum generated by a vehicle. By having a first ANR mode adapted to attenuate a first frequency range and a second ANR mode adapted to attenuate a second frequency range, the hearing protection headset may be suitable to be used in both a mounted and a dismounted configuration.

Accord to a second aspect, the present disclosure provides; a head-worn system comprising the communication system according to the first aspect and related embodiments.

In one embodiment, said headset is configured to automatically detect whether the adaptor element is removably placed between the opening and the flexible seal or not, thereby enabling said headset to automatically detect whether the ANR circuit is set to operate in the first ANR mode or the second ANR mode.

Advantageously, the headset may be configured to automatically determine if the ANR circuit should be det to work according to the first ANR mode or the second ANR mode, such that no manual mode selection or switching are needed to be performed by the user. The headset may automatically apply either the first ANR mode or the second ANR mode making use and functionality of the headset easy and intuitive.

In another embodiment, said headset configured to automatically detect that the headset i worn by a user and in response thereof set the ANR circuit to apply the first ANR mode, when the headset is not worn by the user, set the ANR circuit to apply the the second ANR mode, when the headset is worn by the user.

Advantageously, the headset is capable of detecting that it is worn by a user, such as placed to cover ears of the user and forming a acoustic barrier to protect the hearing of the user from external sounds. This way, the headset may be configured to adapt the active noise cancellation mode applied between a first ANR mode and a second ANR mode. Preferably, the headset is capable of detecting if the headset is worn or not, and if worn, the headset may automatically detect if the earcup cavity volume corresponds to a first cavity volume or a second cavity volume and adjust the ANR mode accordantly. This provides ease of use, as the headset is capable of automatically adapting operation mode and apply the correct ANR mode. It may be beneficial for the ANR circuit to confirm that the headset is worn, such that the first cavity volume or second cavity volume is well defined and sealed from the external environment and reference levels (e.g. From microphones, sensors or speakers) are stabilized before initializing and the first ANR mode or second ANR mode such that unstable performance and feedback loops can be avoided.

In one embodiment, said headset comprises one or more sensors located in and/or on said earcups to detect whether the adaptor element is removably placed between the opening and the flexible seal or not, thereby enabling said headset to automatically detect whether the ANR circuit is set to operate in the first ANR mode or the second ANR mode.

It is advantageous to have one or more sensors capable of detecting and recognizing the presence of the adapter element in relation to the earcups, such that the sensor information may be used by the communication system to automatically set the ANR circuit to apply (e.g. Operate in) a first ANR mode or a second ANR mode, in response to the sensor detecting response. This provide a automatic adaptive behavior of the headset, where he ANR circuit may automatically switch our toggle between a first ANR mode or a second ANR mode dependent of sensor readings rather then cumbersome and time demanding manual adjustment and configuration.

In one embodiment, the one or more sensors is an optical sensor, and/or an electronic sensor, and/or an imaging sensor, and/or an audio sensor, and/or vibration sensor, preferably where the audio and/or vibration sensor is configured to capture audio or vibration, respectively, from the earcup, such as from a signal generator in the earcup, thereby detecting whether the captured audio or vibration matches with the first cavity volume or the second cavity volume.

In one embodiment, said headset is further configured to instruct the ANR circuit to operate in the first ANR mode or in the second ANR mode.

In one embodiment, said headset comprises a processor configured to process output from the one or more sensors and to transmit operation instructions to the ANR circuit to operate either in the first ANR mode or in the second ANR mode.

In one embodiment, said headset further comprises a communication module configured to: connect with at least a first type of peripheral communication device, receive at least one audio signal from the at least the first type of peripheral communication device, and transmit the at least one audio signal to the speaker in the left earcup and/or the speaker in the right earcup.

In it particularly advantageous that the communication system according to the disclosure comprise a communication module, enabling the headset to become a system with both hearing protection capabilities and and communication functionality. The communication module may facilitate the connection to a peripheral communication devices, such as a radio, thereby enabling the user wearing the headset to be in contact with other people via a external communication link, as voice massages received via a connected peripheral communication device may be directed to the users attention via the headset speakers. Such a hearing protecting and communication headset is highly advantageous as a soldier or police officer would only need to carry a single headset having both hearing protection and communication capabilities, as it allows for clear an undistorted communication while protection the users hearing.

By transmitting one or more control-instruction(s) to the first type of peripheral communication device, it is possible to control the first type of peripheral communication device. Simultaneously the communication module may be configured to receive audio signals from the first type of peripheral communication device and play the audio signals to the user via the speakers i the headset. This may be particularly advantageous for personal operating in harsh and demanding environments as peripheral communication devices are used by military and public safety professionals across different agencies for establishing communication channels to share information. In a two-way radio communication setup, two or more individuals or users are equipped with handheld transceivers (e.g. Peripheral communication devices), such as radios which are used to transmit and receive at least voice messages back and forth for establishing clear and undistorted communication in harsh and demanding environments. Certain situations may, for example arise in a harsh environment. For example, users may be required transmit or receive the correct voice communication under stressful circumstances, to make the difference between life and death. Examples first type of peripheral communication devices may be older analog RF Land Mobile Radios (LMR), newer modern digital radios and communication devices like a Mobile Ad Hoc Networking (MANET) radio, tactical smartphone (EUD) or a multiple-input and multiple-output (MIMO) radio etc.

In one embodiment, the processor is part of the communication module.

In one embodiment, said communication module is further configured to transmit one or more control-instruction(s) to the first type of peripheral communication device and wherein said headset comprises a user-interface configured to manually select whether the ANR circuit is set to operate in the first ANR mode or the second ANR mode.

By transmitting one or more control-instruction(s) to the first type of peripheral communication device, it is possible to control the first type of peripheral communication device. Simultaneously the communication module may be configured to receive audio signals from the first type of peripheral communication device and play the audio signals to the user via the speakers i the headset. This may be particularly advantageous for personal operating in harsh and demanding environments as peripheral communication devices are used by military and public safety professionals across different agencies for establishing communication channels to share information. In a two-way radio communication setup, two or more individuals or users are equipped with handheld transceivers (e.g. Peripheral communication devices), such as radios which are used to transmit and receive at least voice messages back and forth for establishing clear and undistorted communication in harsh and demanding environments. Certain situations may, for example arise in a harsh environment. For example, users may be required transmit or receive the correct voice communication under stressful circumstances, to make the difference between life and death. Examples first type of peripheral communication devices may be older analog RF Land Mobile Radios (LMR), newer modern digital radios and communication devices like a Mobile Ad Hoc Networking (MANET) radio, tactical smartphone (EUD) or a multiple-input and multiple-output (MIMO) radio etc. Additionally, in this way, a user may physically interact with the communication system to provide user input to the headset to switch between the first ANR mode and the second ANR mode, which is advantageous as the user wearing the communication system in harsh environment, may be enabled to provide manual instructions to the headset via the user interface in case a specific ANR mode is desired to be applied. This enables the user to overwrite any automatic detecting and force the headset to apply a desired ANR mode, for example in case a vehicle breaks down and a user is changing from a mounted configuration to a dismounted configuration without removing the adaptor element for example.

In one embodiment, said user interface is fixed on said earcup.

In another embodiment, said user interface is removably attached on said earcup via user interface connector comprised by said earcup.

In one embodiment, said headset comprises a user interface connector configured to removably connect a user interface, and wherein said headset is configured such that when the user interface is not removably connected to the user interface connector, then the ANR circuit is set to operate in the first ANR mode, and when the user interface is removably connected to the user interface connector, then the ANR circuit is set to operate in the second ANR mode.

In this way, when the user interface is not removably mounted to the user interface connector, then the ANR circuit may be set to operate in the first ANR mode as a the default mode. Then he user interface is connected, it might be recognized by headset when mounted to the user interface connector, which then instruct the ANR circuit to apply the second ANR mode. Thus, the connection of the user interface to the headset (e.g. Via the user interface connector) may be used as a proxy detecting mechanism for instructing the ANR circuit to switch from applying the first ANR mode to instead apply the second ANR mode. This is advantageous, as the headset do not need to comprise additional sensors, drawing power, as the connection of the user interface may be associated with the earcup cavity volume corresponding to the second cavity volume

In one embodiment, the user interface comprises one or more tangible controls that are manually operably by the user.

It may be particular advantageous to have tangible controls on the user interface when used by personal in harsh environments, as the tangible controls should be manually operable by the user under difficult and stressful circumstances to enable clear and undistorted communications, even while wearing thick gloves or being in darkness such that the structure and design of the controls are tangible to enable unambiguously manual operation.

In one embodiment, one of said tangible controls is a switch.

In one embodiment, the switch allows the communication module to transmit the one or more control-instructions to the at least first type of peripheral communication device.

In one embodiment, the one or more control-instructions is/are in the form of Push-to-Talk (PTT) signals and wherein the at least first type of peripheral communication device in the form of a radio.

In one embodiment, when in the first ANR mode, artificial hearing is enabled, and when in the second ANR mode, artificial hearing is disabled.

In one embodiment, the first ANR mode is the same as the second ANR mode.

In one embodiment, wherein the left earcup and the right earcup each comprise an ambient microphone configured to capture the external sounds, and wherein the communication system further comprise a processor configured to process the captured external sound signals and provide the artificial hearing by transmitting the processed captured external sound signals to the user via the corresponding left and the right earcup, thereby providing the user the ability to hear the surroundings when wearing the headset.

Advantageously, the headset i capable of providing artificial hearing such as "hear through" enabling external sounds that the user might want to hear to be registered and reproduced by speakers inside the headset. This function providing may provide effect of making the headset "transparent" in terms of listening to the surroundings. This is highly advantageous for a user operating in a dismounted configuration, as the hearing sense is important to maneuver and react to unexpected situations, such as an enemy helicopter approaching or alike. Oppositely, artificial hearing is unwanted when a user is operating in a mounted configuration as the external sounds are dominated by vehicle noise which should be attenuated as much as possible. By enabling the headset to provide artificial hearing when the hearing protection headset operates in the first ANR mode or disable artificial hearing when the hearing protection headset is operating in the second ANR mode, the communication system according to the present disclosure may be used by users working in harsh and demanding environments in both a dismounted configuration and a mounted configuration. Artificial hearing advantageously comprise a processing step of the captured external sounds before directing the external sound signals to the speakers. This processing step may comprise limiting the overall amplitude of the captured external sound signals emitted by the speaker units so as to protect the user's hearing from overloudly sound pulses from gun fire, explosions. Advantageously, the artificial hearing is processed in real-time, such that no substantial delay is perceived by the user when the artificial hearing is enabled.

In one embodiment the left earcup and the right earcup each comprises an internal (monitor) microphone configured to capture internal sound from inside the respective earcup when worn by a user, and wherein the communication system further comprise a processor configured to provide the ANR functionality by, e.g. or preferably continuously, monitoring the respective captured internal sound and generating a respective inverse sound signal that is in counter phase with the respective captured internal sound.

Accordingly, in view of the above preferred embodiments, there is provided a communication system for communicating in a harsh environment that is configured to adapt to a first situation with low noise and to a second situation with high noise.

### BRIEF DESCRIPTION OF THE DRAWINGS

Embodiments of the invention will now be described in more detail. Various embodiments of the systems and/or the methods according to the different aspects as disclosed herein will be described in connection with the appended drawings, in which:
FIG. 1A schematically illustrates an example of a user 100 in a dismounted configuration 114 wearing a communication system 102 configured to be worn by a person operating in a harsh and demanding environment, in communication with other connected units via a peripheral communication device 110.
FIG. 1B schematically illustrates an example of a user 100 in a mounted configuration 116, wearing a communication system 102 configured to be worn by a person operating in a harsh and demanding environment, in communication with other connected units via a peripheral communication device 110.
FIG. 2A schematically illustrates an example of a hearing protection headset 104 to be worn by a person 100 operating in mounted dismounted configuration 114 to be used in a harsh and demanding environment, according to embodiments of the disclosure.
FIG. 2B schematically illustrates an exploded view of the right earcup 200b of a communication system 102 embodied as a hearing protection headset 104, showing the internal components.
FIG. 2C schematically illustrates an exploded view of the left earcup 200a of a communication system 102 embodied as a hearing protection headset 104, showing the internal components.
FIG. 2D schematically illustrates an example of a hearing protection headset 104 comprising adaptor elements to be worn by a person 100 operating in mounted configuration 116 to be used in a harsh and demanding environment, according to embodiments of the disclosure.
FIG. 3 schematically illustrates block diagram of a communication system 102 according to embodiments of the disclosure.
FIG. 4 schematically illustrates an example of communication system 102 comprising a left connector interface 216a and a right connector interface 216b in connection with a communication module 106 according to the disclosure.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiment even if not so illustrated, or if not so explicitly described.

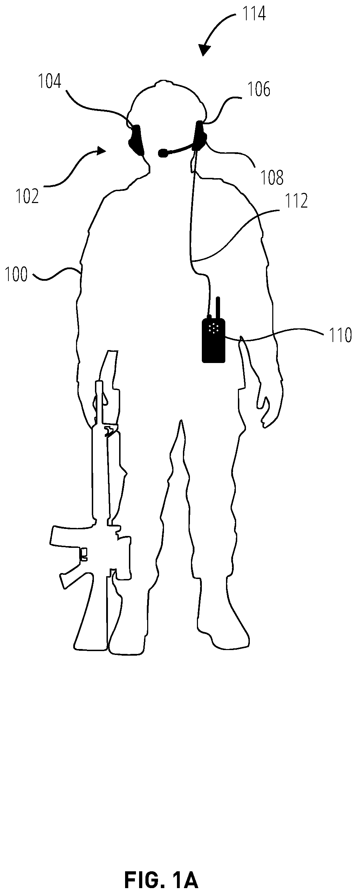

Various aspects and embodiments of a communication system as disclosed herein will now be described with reference to the figures FIG. 1A-FIG. 4.

In one embodiment, the communication system is configured to be worn by a person operating in a harsh and demanding environment, in communication with other users connected via a peripheral communication device. In a two-way radio communication setup, two or more individuals or users are equipped with handheld transceivers (e.g. Peripheral communication devices), such as radios. Each peripheral communication device may operate on designated frequencies or talk group, enabling the transmission and reception of voice messages over radio waves (i.e. Radio frequency). When one user activates their radio, their voice may be converted into electrical signals, which are then modulated into radio waves and transmitted via the peripheral communication device. A second peripheral communication device, tuned to the same frequency or talk group, may receive these waves, demodulate them, and convert them back into audible sound. This radio frequency (RF) communication link may allow for clear and undistorted communication between users over varying distances. Hence, in one embodiment, the peripheral communication device 110 is configured to establish person to person voice communication, audio and data communication links with other peripheral communication devices.

In one embodiment a user may wear a wearable communication system comprising a circumaural hearing protection headset having a left earcup and a right earcup both configured to attenuate external sounds from the harsh environment, such as loud pulse noises from gunfire, explosions or loud vehicle noises, thereby protecting the hearing of the user.

In another embodiment, the communication system comprises a communication module configured to connect with a first type of peripheral communication device and receive at least one audio signal from the first type of peripheral communication device.

In some embodiments, the communication module may be configured to transmit the at least one audio signal to a speaker in the left earcup and/or a speaker in the right earcup.

In one embodiment, the communication system is integrated into a single hearing protection headset.

In one embodiment the communication system may comprise a hearing protection headset, in the form of a pair of earcups adapted to be situated over the ears of the user when worn, a communication module, configured to establish and maintain one or more communication links via a connected first type of peripheral communication device and ANR circuit configured to apply a first ANR mode or a second ANR mode

This advantageously enables the hearing protection headset to be used by a user in a so-called "dismounted configuration" or a "mounted configuration". A dismounted configuration refers to a situation where the user may maintain a communication link (e.g. person-to-person voice communication, audio and/or data communication links) with one or more remotely located other users via their respective communication systems and peripheral communication devices and wherein the person is primarily operating outside a vehicle, such as a helicopter, tracked vehicle, boat etc. Hence, being "dismounted" refers to a user operating in a harsh and demanding environment free to move around in open air while being able to maintain a communication link. In the dismounted configuration, the user may occasionally be exposed to harmful external sounds.

Examples of users operating in the dismounted configuration may be infantry soldiers who operate in some of the most physically and mentally demanding environments, often exposed to harsh weather, rough terrain, and constant danger. They march for miles carrying heavy gear, facing exhaustion, hunger, and extreme temperatures. On the battlefield, they endure relentless noise, chaos, and the threat of ambushes, snipers, or explosives. Combat requires quick thinking, teamwork, and split-second decisions under life-threatening conditions. Whether fighting in dense jungles, urban ruins, or open deserts, infantry must adapt rapidly, relying on rigorous training and resilience.

A mounted configuration refers to a situation where the user may maintain a communication link (e.g. person-to-person voice communication, audio and/or data communication links) with one or more remotely located other users via their respective communication systems and peripheral communication devices wherein the person is primarily operating inside a vehicle, such as a helicopter, tracked vehicle, boat etc. In the dismounted configuration, the user may be consistently exposed to extreme and harmful external sound levels. Hence, being "mounted" refers to a user operating in a harsh and demanding environment inside or confined by a vehicle, such that the user is not free to move far away from the vehicle while still maintaining a communication link.

Examples of users operating in the dismounted configuration may be soldiers in armored forces and artillery units who operate in intense, high-stakes environments. Armored crews work in confined, heavily armored vehicles, enduring extreme heat, noise, and the constant threat of anti-tank weapons. They must navigate rough terrain, coordinate with infantry, and make rapid decisions under fire. Artillery crews operate in exposed or fortified positions, enduring deafening blasts and harsh weather as they load and fire massive weapons. They work under pressure to provide precise, long-range support while remaining vulnerable to counterattacks.

FIG. 1A schematically illustrates an example of a user 100, being a soldier, operating in a mounted configuration 116 wearing a communication system 102 configured to be worn by a person operating in a harsh and demanding environment, in communication with other users (not shown) via a first type of peripheral communication device 110. The user 100 is wearing a communication system 102 integrated into a hearing protection headset 104 and one type of radio 110 connected to the communication system 102 via a cable 112. The hearing protection headset 104 may comprise a user interface 108, such that the user may control and operate the first type of peripheral communication device 110 Alternatively, the cabled connection may be replaced by suitable wireless connection. The user 100 may carry and use one or different types of peripheral communication devices 110 operably connected to the communication system 102 for establishing person to person voice communication, audio and data links with other remote users as previously described.

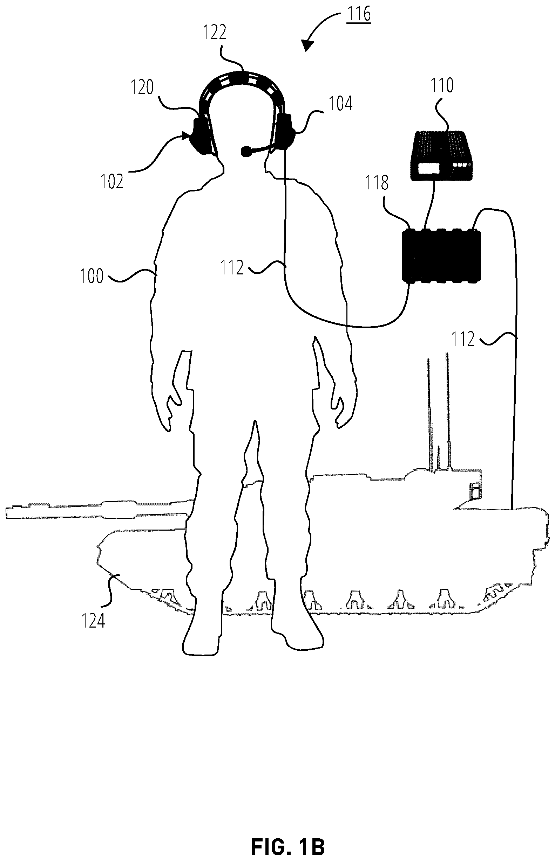

FIG. 1B schematically illustrates an example of a user, being a Combat Vehicle Crewman (CVC), operating in a mounted configuration 116 in an armored vehicle 124, like a Leopard battel tank. The user 100 is wearing a communication system 102 configured to be worn by a person operating in a harsh and demanding environment, in communication with other users (not shown) via a first type of peripheral communication device 110. The user 100 is wearing a communication system 102 integrated into a hearing protection headset 104 including a set of adapter elements 120 and fixed to an impact liner 122 waring style. The hearing protection headset 104 may be connected to a vehicle mounted radio 110 via a communication hub 118 such as an intercom device via a cable 112.

### Peripheral communication device

In one embodiment aa first type of peripheral communication device is a mobile communication device.

In one embodiment a first type of peripheral communication device may be a mobile communication device configured to be carried around and provide a wireless communication link (e.g. A radio frequency (RF) communication link) to another peripheral communication device. Preferably, a first type of peripheral communication device may be understood as a device for providing voice and/or data transmission wirelessly over long distances using radio frequency bands or other electromagnetic wave bands. In one embodiment, a first type of peripheral communication device may be a handheld 2-way radio for providing voice and data communication in the VHF and UHF bands using different waveforms. Thereby, a first types of peripheral communication device may offer secure and reliable communication in various harsh operational environments. Examples of peripheral communication devices may be an L3Harris^{®} Falcon III AN/PRC-152A that provides simultaneous voice, video and high-speed data in a highly portable form factor for dismounted use which provides interoperable communication in multiple frequency bands and supports voice and data transmission. Examples of vehicular integrated peripheral communication devices may include the Thales SYNAPS-V 2-channel V/UHF radio and the L3Harris Falcon III AN/PRC-117G Multiband Networking Manpack Radio. Other examples of a first type of peripheral communication devices may be a specialized radio system to be used in harsh and demanding environments by first responders or military personal, such as the analog Motorola GP340 or the digital DP4000E portable two-way radios, the TrellisWare Shadow 950/900 tactical radio, or the Barrett PRC-2090 HF tactical radio, each offering long-range communication capability in the HF band for dismounted operations. Yet other types of peripheral communication devices may be a chest mounted Tactical Display Unit (TDU) or End-User-Device (EUD), such as a Samsung Galaxy S23 Tactical Edition or Samsung XCover6 Pro Tactical Edition smart phones with a 6. 1-inch touch screen or alike, supporting conventional cellular capabilities as well as secure tactical communication, software applications etc. It may be advantageous for the person operating in a harsh and demanding environment to carry a chest mounted Tactical Display Unit (TDU) or End-User-Device (EUD) for providing visual information to the user via applications like the Android Team Awareness Kit (ATAK) and the Battlefield Assisted Trauma Distributed Observation Kit (BATDOK) which advantageously enhances real-time situational awareness for the user through collection and display of information using functions like as mapping, messaging, and geofencing. This enables all team members (e.g. Multiple users) in a group to individually access relevant information simultaneously on a single screen. Thus, the different types of peripheral communication devices provide reliable communication links between a user, team members, units and central command centers to facilitate coordination and situational awareness on the battlefield or in emergency situations.

### Hearing protection and communication headset

In a preferred embodiment, the communication system for communicating in a harsh environment, comprise a hearing protection headset to be worn by a user in the harsh environment, said hearing protection headset comprising: a left earcup and a right earcup configured to attenuate external sounds from the harsh environment. Each of said earcup may comprises; a speaker unit; a cavity with a opening adapted to accommodate the ear of the user when worn and a flexible seal around the periphery of the opening to form an ear opening, wherein the flexible seal is configured to acoustically seal the cavity when worn by the user. Each of said earcup and flexible seal defines a first cavity volume, wherein the cavity first cavity volume contributes to providing passive sound attenuation, whereby each earcup is configured to attenuate external sounds from the harsh environment.

Further, and also according to the present disclosure, the adaptor element has a central void and a rim having a thickness such that the central void defines a void volume, and wherein the adaptor element is configured to be removably placed between the opening and the flexible seal such that the rim rest against the periphery of the opening, thereby effectively increasing the first cavity volume to a second cavity volume defined by the first cavity volume and the void volume, thereby increasing the passive sound attenuation.

Accordingly, the adapter elements increase the cavity volume and may themselves adapt the headset to, for example CVC operation (e.g. See Dismounted configuration 114 in FIG. 1B) in extreme high noise.

However, when the cavity volume is increased, the headset also enlarged in size. The enlarged size may interfere with several types of ballistic helmets worn by military persons, for example when a military person is not inside the vehicle. Accordingly, in situations where the military person is outside the vehicle (e.g. See dismounted configuration 114 in FIG. 1A), the military person may desire to decrease the size of the headset. The presently disclosed headset provides for such a solution, where the solution is to remove the adaptor element(s). This also has the benefit of decreasing the weight of the headset, thereby making it more comfortable for the person to wear the headset, particularly when the person is walking or running. As described, when the adaptor element is removed, at least the passive noise reduction is reduced. This is further advantageous because when outside the vehicle, there may not be a need for high or extreme noise reduction. On the contrary, there may be a need for low noise reduction, for example such that the military person can hear enemies and other events in the environment. Accordingly, the presently disclosed headset provides several advantages in different situations.

As described there may be a first situation where the military person is outside a vehicle (e.g. A dismounted configuration 114 in FIG. 1A), and a second situation where the military person is inside the vehicle (e.g. Mounted configuration 116 in FIG. 1B).

As described above, the adapter adaptor elements increase the cavity volume and may themselves adapt the headset to CVC operation in extreme high noise. The reason for this is as follows. Various physical parameters such as mass, cushion material and cavity size may impact the passive sound attenuation characteristics of headset additionally parameters for the overall performance may be the presence of porous absorption material inside the ear-cup and leakage between the cushion and the user. However, the sound pressure inside the earcup is mainly due to the compression and rarefaction of the volume of air enclosed between the ear and the ear cup. It is generally known from thermodynamics that the passive sound attenuating effect of an ear-cup is dependent on the volume inside the ear-cup cavity. Hence, an increase in volume of the enclosure causes a decrease in pressure inside the cavity, thus an increased sound attenuation effect.

The noise profile inside military vehicles is typically characterized by very high overall sound pressure levels, often exceeding 100-120 dB and dominant in the low-frequency spectrum, particularly from the drive train and track links around 100-200 Hz. Such a noise environment may be challenging, potentially affecting communication, causing stress, and risking hearing damage if proper precautions are not taken. Accordingly, the increase in volume, as described above, may not always be sufficient to provide optimal sound reduction, for example if the noise is as inside a military vehicle. For optimal sound reduction, active noise reduction (ANR) may be introduced, such as described above in the preferred embodiments and throughout the disclosure.

In one embodiment, the hearing protection headset comprises an active noise reduction (ANR) circuit, configured to attenuate the external sounds from the harsh environment by generating anti-noise signals, via said speaker units, that destructively interfere with the external sound, wherein the ANR circuit is configured to operate in a first ANR mode or a second ANR mode.

The first ANR mode may accordingly be suitable for the first situation such as a dismounted configuration (e.g. See FIG. 1A), whereas the second ANR mode may be suitable for the second situation, such as a mounted configuration (e.g. See FIG. 1B).

In the situation where the military person is outside the vehicle, and thus where the headset is in the first ANR mode, the first mode may be referred to as a dismounted mode (e.g. Then the user is operating in the dismounted configuration 114). The dismounted mode refers to the situation where the adaptor element(s) is/are dismounted from the hearing protection headset. Likewise, in the situation where the military person is inside the vehicle, and thus where the headset is in the second ANR mode, the second mode may be referred to as a mounted mode (e.g. When the user is operating in the mounted configuration 116). The mounted mode refers to the situation where the adaptor element(s) is/are mounted to the hearing protection headset.

In view of the above, the hearing protection headset according to the disclosure may be configured to be used in a military vehicle for providing both hearing protection and communication capabilities. The hearing protection characteristics, i.e. Second ANR mode, may be adapted to a CVC configuration.

In some embodiments, there is provided a head-worn system comprising the communication system. Other embodiments of the presently disclosed communication system and headset will be described in the following.

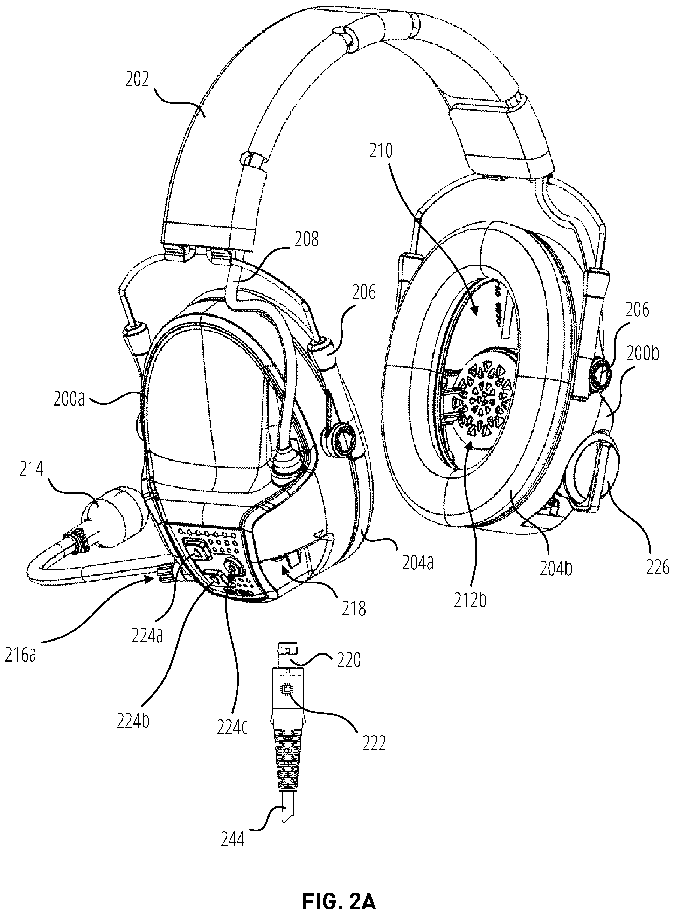

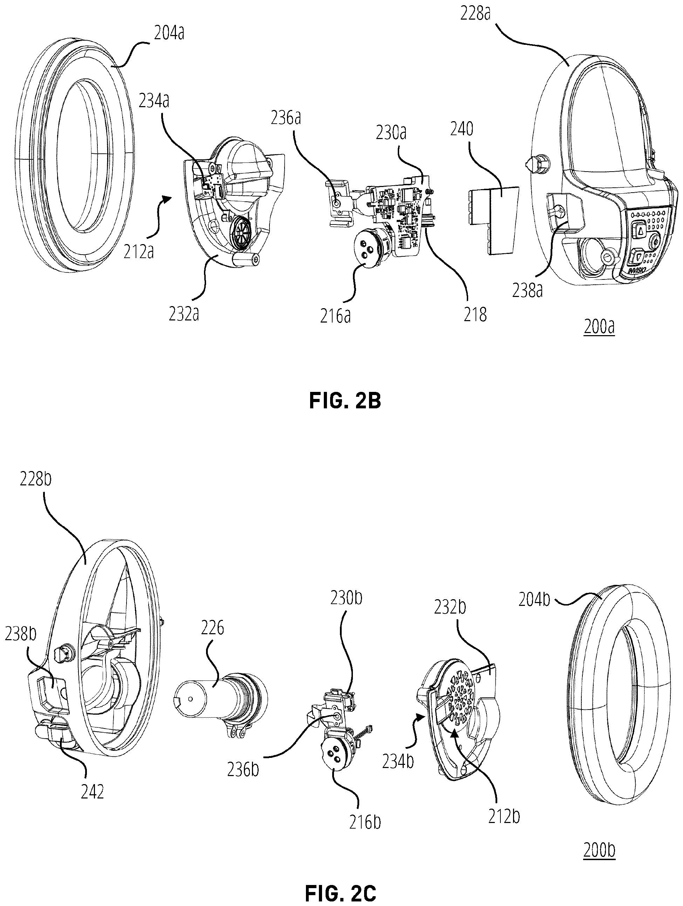

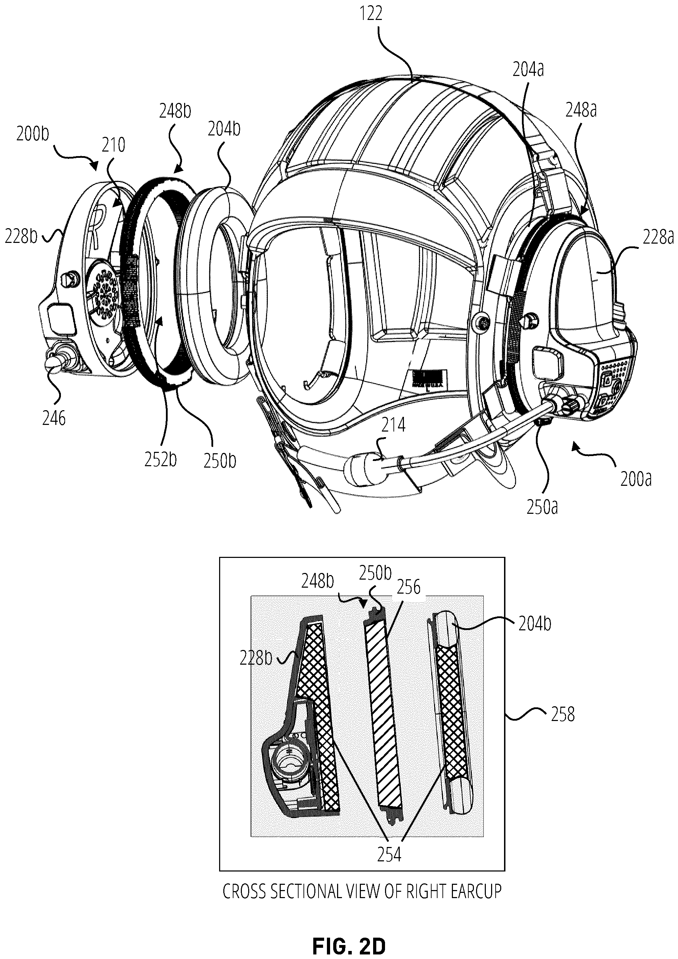

FIG. 2A- FIG. 2D schematically illustrates an example of a communication system 102 to be worn by a person 100 operating in a harsh and demanding environment, embodied in a hearing protection headset 104. FIG. 2B shows examples of the individual components comprised in the left earcup 200a and FIG. 2C shows examples of the individual components comprised in the right earcup 200b in a sequential special arrangement to highlight some internal components and their structural and functional relationship. FIG. 2D shows an example of the hearing protection headset 104 comprising an adapter element 120.

In one example illustrated in FIG. 2A, the hearing protection headset 104 may include a left earcup 200a and a right earcup 200b and a headband 202. The headband 202 may couple the left earcup 200a with the right earcup 200b both physically, via a coupling arrangement 206, and electronically via a cup-to-cup cable 208. The headband 202 can be arched, such as to extend over the top of a user's 100 head while the headset is in use (e.g. worn by the user 100). The headband 202 may be flexible, such as allowing the user 100 to spread the left earcup 200a from the right earcup 200b when the user 100 is putting on the hearing protection headset 104. The headband 202 may include padding, such as to at least partially conform to the user's head and increase the user's comfort. Both the left earcup 200a and right earcup 200b may be configured to fit around a user's 100 ear and be disposed on the side of a user's head while in use. Both earcups 200a, 200b may include a flexible seal (e.g. A left cushion 204a and a right cushion 204b) adapted to extend around the user's 100 ear. The flexible seals 204a, 204b may be flexible and able to conform to the user's 100 head and provide an acoustic seal between the earcups 200a, 200b and the user's 100 head, such as to attenuate the amount of noise or sound waves that reach the user's 100 ear, to at least partially protecting the user's ear from external noises. Thereby providing passive hearing protection. Thus, the left earcup 200a in combination with the left cushion 204a and the right earcup 200b in combination with the right cushion 204b may each respectively define a first cavity volume 210 for each of the user's 100 ears when wearing the hearing protection headset 104.

Even though not explicitly seen from FIG. 2A, the hearing protection headset 104 may contain structures (e.g. See FIG. 2B and FIG. 2C), features and functionalities at described at least in part in both document US20220217459A1 and US 11968493B2 (hereby incorporated by reference in its entirety). The hearing protection headset 104 may include a speaker unit, such as a left speaker unit 212a located in the first cavity volume of the left earcup 200a and a right speaker unit 212b located in the first cavity volume 210 of the right earcup 200b. Both speaker units 212a, 212b being directed towards the ears of the user 100 when wearing the headset 104 such that a sound signal can be played via one or both of the speaker units 212a, 212b enabling the user 100 to receive auditive information when waring the hearing protection headset 104.

In some embodiments, hearing protection headset 104 may include a transmission (Tx) microphone, such as boom microphone 214 integrated into a flexible arm or rod adapted to position the microphone relatively close to the mouth of the user 100.

In the illustrated example in FIG. 2A, the boom microphone 214 may be configured to pick up communications from a user 100, such as recording a voice signal to be transmitted using radio frequency to a remote receiver, such as via a connected peripheral communication device 110. In a preferred embodiment, the boom microphone 214 may be releasably connected to a connector interface (e.g. 216a or 216b) on the headset 104.

In various embodiments the hearing protection headset 104 may include individual connector interfaces, such as a user interface connector and/or microphone connector (e.g. A left connector interfaces 216a and a right connector interface 216b) on each of the two earcups 200a, 200b such that a boom microphone 214 may be positioned on the left earcup 200a and/or on the right earcup 200b. This is advantageous as the boom microphone 214 may thereby be mounted depending on user's 100 preference. It may be advantageous to mount the boom microphone 214 opposite of the user's 100 preferred side to handle a weapon, as the boom microphone 214 otherwise may conflict with aiming and shooting movements requiring the gunstock to rest against the user's 100 shoulder (e.g. on the preferred side / trigger finger side). Thereby allowing ambidextrous placement of the boom microphone 214.

In another embodiment, the hearing protection headset 104 may contain a receptacle interface 218 for releasably connecting a peripheral communication device 110 via a cable 112 containing a connector or plug 220. The receptacle interface 218 may allow the user 100 to connect an external first type of peripheral communication device 110 into the communication system 102.

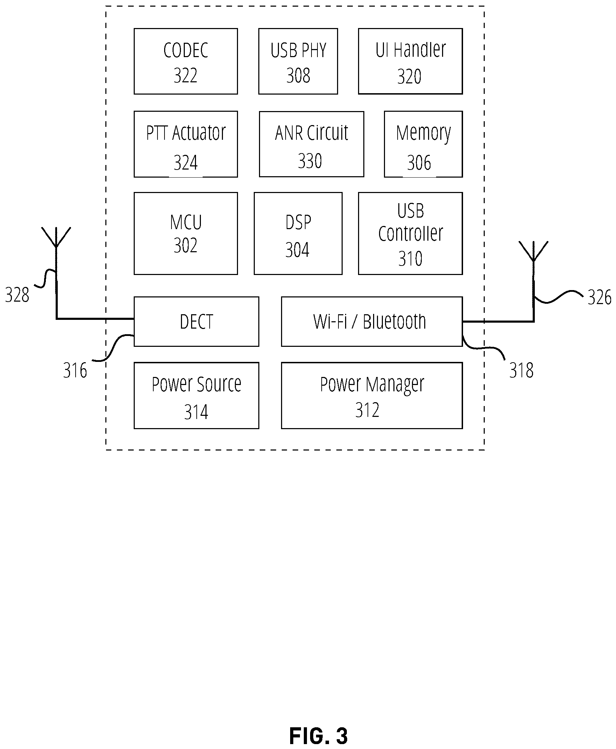

In the illustration example in FIG. 2A the hearing protection headset 104 comprising the communication module 106 additionally comprises a receptacle interface 218, configured to releasably connect a peripheral communication device 110 such as an AM/FM radio, a two-way radio, intercom and/or a cell phone as described elsewhere via a cable 112 and a connector 220. The receptacle interface 218 may be in contact with a communication module 106 (e.g. see FIG. 3) comprised by one or both of the earcups 200a, 200b and configured to connect and exchange control-signals, control-instructions, audio signals and other information and data with the peripheral communication device 110.

In some embodiments, when a peripheral communication device 110 is connected to the communication module, coupling data associated with the peripheral communication device 110 may be obtained by the communication module 106. In one embodiment, such coupling data may contain device type information specifying the type of peripheral communication device 110 (e.g. A first type of peripheral communication device or a second type of peripheral communication device). In the situation where a peripheral communication device 110 may be connected via a connector 220 configured to interface with the receptacle interface 218 on the headset 104, information and functional settings (e.g. Control-instruction(s) and communication protocol(s)) or information thereof) may be transmitted via the connection interface e.g. or preferably as described at least in part in EP2845 115B1 (hereby incorporated by reference in its entirety) from a microchip (e.g. cable chip 222) embedded or located in the connector 220 (or alternative embedded or located in the cable 112 of the connector 220 or elsewhere).

In one embodiment, the cable chip 222 may be a microchip comprising an embedded memory configured for storing programmable data representing code, settings, instructions, and/or other data.

In some embodiment, one or both of the earcups, such as the left earcup 200a and/or the right earcup 200b may contain a user interface 108 allowing the user 100 to provide manual user inputs for controlling the connected peripheral communication device 110, adjusting the ANR modes (e.g. Between a first ANR mode and a second ANR mode) and other functions of the 104 e.g. perform man-machine interactions (MMI). For example, as shown in FIG. 2A the user interface 108 is displayed in the form of a rubber keypad, with three tangible controls elements in the form of push-buttons (224a-224c), such as an up-button 224a and down-button 224b" and a middle-button 224c. In the present example, the user interface 108 may be configured to allow the user 100 to easily interact with the buttons (224a-224c) based on touch and feel rather than visual confirmation which is advantageous as the user interface 108 may be positioned outside the users field of view, such as the side of head (e.g. on the headset 104 when worn).

In an alternative embodiment, the tangible control element may be a multi position rotatable knob, a toggle switch or mini joystick configured to support multiple operation states such as a momentary toggle switch, where the pin returns to a default position when released or may stay in one state until manually changed.

In a preferred embodiment, the hearing protection headset 104 may contain a battery compartment 226 for holding a power source such as a single use or multiuse battery for powering the communication system 102. It may be advantageous to configure the communication system 102 to use standard alkaline batteries such as an AA or AAA batteries or alike, as such batteries are easy to procure and replace around the world when depleted, thereby securing a reliable power source in demanding and harsh environment.

FIG. 2B shows an example of the individual components comprised in the left earcup 200a in a sequential special arrangement to highlight some internal components and their structural and functional relationship. The left earcup 200a containing a left primary housing 228a configured to accommodate, fixate and seal the internal components. The housing 228a may be made in acoustically attenuating material such as rugged plastic or polymer material, preferably reinforced with carbon for reducing the weight and increasing the strength and durability while having good acoustically damping properties. A user interface 108 may be located on the external surface of the housing 228a, such that it is easy to always reach for the user 100. Inside the housing 228a, a primary printed circuit board (PCB) 230a may be mounted and sealed from the external environment by the left speaker unit assembly 232a containing the left speaker unit 212a. The left cushion 204a may be adapted to engage with the left primary housing 228a via a snap lock, enabling the user 100 to easily replace the cushions (e.g. 204a and 204b) when worn down or to mound an adapter element 120 in-between the left primary housing 228a and the left cushion 204a without using any tools (e.g. See FIG. 2D). As previously mentioned, the left cushion 204a may form a compatible acoustic seal between the user 100 and the left primary housing 228a, such that the cushion 204a and housing 228a together form a left earmuff having a first cavity volume for passive sound attenuation providing hearing protection on the left ear of the user 100 when worn. The left speaker unit assembly 232a may be configured to hold the left speaker unit 212a, being a transducer or loudspeaker for transforming an electronic signal to an acoustic signal such that voice and sound are transmitted electronically via the hearing protection headset 104 and played to the user 100 when wearing the hearing protection headset 104.

In one embodiment, the left speaker unit assembly 232a may additionally a water and dust tight seal around the internal electronic components (e.g. the primary PCB 230a) enabling the hearing protection headset 104 to have at least a IP68 rating specifying water resistant in fresh water to a maximum depth of 1.5 meters for up to 30 minutes, and are protected from dust, which is preferred when the headset 104 is adapted to be used in harsh and demanding environments.

In one embodiment the left speaker unit assembly 232a may additionally accommodate a left monitor microphone 234a and related circuitry.

In one embodiment the left monitor microphone 234a may be a transducer for converting an acoustic response into an electric signal.

In another embodiment the left monitor microphone 234a may be positioned and exposed to the internal cavity inside the left earcup 200a. Thereby configuring the left monitor microphone 234a to monitor the sound environment inside the left earcup cavity (e.g. First cavity volume 210 or second cavity volume.

In another embodiment a left ambient microphone 236a may be connected to the primary PCB 230a and mounted on a rail for interlocking and sealing with the housing 228a.

In one embodiment the left ambient microphone 236a may be a transducer for converting an acoustic response into an electric signal and positioned in a forward-facing position on the left earcup 200a. Thus, the left ambient microphone 236a may monitor the external sound environment surrounding the left earcup 200a.

In the example shown in FIG. 2B the left primary housing 228a may contain a left recess 238a on the external surface around the active sensing area of the left ambient microphone 236a for holding a wind filter (not shown), such as a foam or porous material for mechanically damping or removing turbulent noise from wind etc. A left connector interface 216a may additionally be connected to the main primary PCB 230a. The left connector interface 216a contains three receptor terminals for connecting to the boom microphone 214 or a toggle switch 246.

In a preferred embodiment, the left connector interface 216a is configured to releasably connect a first type of accessory device and/or a second type of accessory device.

In one embodiment the electronic receptacle interface 218 and corresponding circuitry may additionally be connected to the primary PCB 230a. The electronic receptacle interface 218 may be configured for connecting to one or more different types of peripheral communication devices 110 and exchange electronic signals, via a plurality of connector pins.

In one embodiment, the primary PCB 230a contains one or more processors, configured to execute computer readable instructions causing the different components to perform the desirable actions and functions to provide the capabilities of the communication system 102 according to the disclosure as explained in more detail in relation to FIG. 3, and throughout the disclosure. An electromagnetic compatibility (EMC) shield 240 may be arranged to cover the primary PCB 230a configured to shield the user 100 and other internal components from potential electromagnetic radiation which may cause noisy signals.

In one embodiment, the right earcup 200b may contain similar components and related embodiments as represented in relation to the left earcup 200a (e.g. In FIG. 2B) which is performing the substantially equivalent tasks as the left counterparts, just related to the right-hand side of the user 100. An example of the right earcup 200b is shown in FIG. 2C displaying the internal components, such as the right primary housing 228b and wind filter recess 238b, right cushion 204b, right speaker unit assembly 232b, right monitor microphone 234b (not explicitly shown), right ambient microphone 236b and right connector interface 216b, such that the right cushion 204b and right primary housing 228b together form an right earmuff having a first cavity volume for passive sound attenuation providing hearing protection on the right ear of the user 100 when worn.

In one embodiment the right earcup 200b may contain a battery compartment 226 configured to accommodate a battery type power source for powering at least the headset 104. The right primary housing 228b may contain a cover cap 242 for covering the left connector interface 216a not used for the boom microphone 214 (or any other type of accessory device, e.g. See FIG. 2D) such that the headset 104 maintains the IP68 rating. The right earcup 200b may additionally contain a secondary PCB 230b connected to the electronic components in the right earcup 200b and connected to the primary PCB 230a and other components in the left earcup 200a via the cup-to-cup cable 208. The secondary PCB 230b may contain one or more additional processors and circuitries operably coupled with the primary PCB 230a for enabling the components of the headset 104 (i.e. in both earcups 200a and 200b) to function together and provide the capabilities of the communication system 102.

In one embodiment, the primary 230a and/or secondary PCB 230b may thus constitute at the communication module 106 of the communication system 102, explained in more details in relation to FIG. 3 and throughout the disclosure.

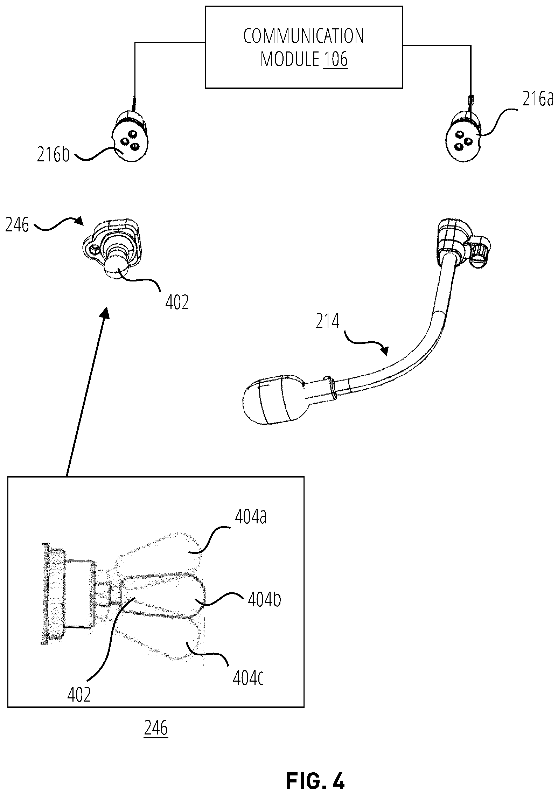

In the example illustrates in FIG. 2D the hearing protection headset 104 as shown in FIG. 2A has been adapted to CVC operation (e.g. Mounted mode). The illustration in FIG. 2D shows a different configuration of the head-worn system (e.g. Hearing protection headset 104 in FIG. 2A specifically adapted for CVC operation, such as in an armored vehicle (e.g. Mounted configuration 116 in FIG. 1B). The extreme noise levels inside these vehicles necessitate the use of hearing protection specifically configured for the CVC operation, and the presently disclosed communication system 102 may thus be adapted for CVC operation. In FIG. 2D, the right earcup 200b is displayed in an exploded view to illustrate the arrangement of elements whereas the left earcup 200a is displayed in a collapsed configuration to illustrate the left earcup 200a arrangement in the assembled state. A CVC typically wears a specialized Combat Vehicle Crewman's (CVC) helmet or impact liner 122 specifically adapted to integrate high noise hearing protection. Each of the left earcup 200a and right earcup 200b comprise an adaptor adapter element 120, in the form for a left adapter ring 248a and a right adapter ring 248b. Each adapter element 248a, 248b has a central void 252a, 252b and a rim 248a, 248b having a thickness such that the central void 252a, 252b defines a void volume 256. The left adapter ring 248a is configured to be removably placed between the opening of the right primary housing 228b and the right cushion 204b (i.e. flexible seal) such that the right rim 250b rest against the periphery of the opening of the left primary housing 228a, thereby effectively increasing the first cavity volume 210 (e.g. See FIG. 2A) to a second cavity volume. The sub figure 258 in FIG. 2D shows a cross-sectional view of the right earcup 200b, displaying the right primary housing 228b, the right adapter rings 248b and the right cushion 204b in an exploded arrangement. The cavity of the right primary housings 228b in combination with the cavity of right cushion 204b (illustrated by the crossline shaded area 254) defines a first cavity volume 210. When the adapter element is mounted, the void volume of the right adapter rings 248b (illustrated by the diagonal line shaded area 256 ) may increase the total volume of the right earcup 200b to a second cavity volume being the combined area of the first cavity volume (e.g. first volume 254) and the void volume 256, such that the left cushion 204a, left adapter ring 248a and left primary housing 228a together form an left earmuff having a second cavity volume for improved passive sound attenuation (i.e. compare the first cavity volume) providing hearing protection on the right ear of the user 100 when worn thereby increasing the passive sound attenuation. The change in total volume of the left earcup 200a, is equivalent to the example provided for the right earcup 200b even though not explicitly shown in FIG. 2D, just related to the cavity of the left earcup 200a, the cavity of the left cushion 204a and the void volume of the left adapter ring 248a such that the right cushion 204b, right adapter ring 248b and right primary housing 228b together form an right earmuff having a second cavity volume for improved passive sound attenuation (i.e. Compared to the first cavity volume) providing hearing protection on the right ear of the user 100 when worn. Now referring to the main illustration in FIG. 2D, the rim (250a, 250b) of both the left adapter ring 248a and the right adapter rings 248b each comprise a mechanical fixation structure for interlocking with the impact liner 122 to securely attach the earcups 200a, 200b, such that the adapter elements provide the additional functionality (in addition to the volume increase) of an earcup attachment arrangement. The rims (e.g. 250a, 250b) of the adapter elements (e.g. The left 248a and right adapter ring 248b) may be designed with a snap-lock mechanism, such that the user may detachably mount the adapter elements to the earcups 200a, 200b and the impact liner 122 without the need of any tools. A toggle switch 246 is mounted to the right connector interface 216b and a boom microphone 214 is mounted to the left connector interface 216a as explained in more details in relation to FIG. 4 and elsewhere.

### Communication module

In one embodiment, said headset further comprises a communication module configured to: connect with at least a first type of peripheral communication device, receive at least one audio signal from the at least first type of peripheral communication device, transmit the at least one audio signal to a speaker in the left earcup and/or a speaker in the right earcup, and transmit one or more control-instruction(s) to the first type of peripheral communication device.

In this manner, a CVC may for example obtain clear and undistorted communication to operate effectively during missions. Examples of communication may be intra-vehicle communication to establish clear and uninterrupted communication between crew members inside the vehicle, and external radio communication for communicating with other vehicles, ground troops, and command centers.

In most embodiments, the communication module comprise a processor.

In some embodiments, the communication module comprises one or more processors, such as a DSP and/or an MCU.

In one embodiment, the communication module may contain a main control unit (MCU) such as a microcontroller system (e.g. ST from STMicroelectronics) such as an ARM processor core based on a Reduced Instruction Set Computing Architecture (RISC) and/or an FPGA processor or similar, for executing instructions, controlling other units, and/or performing complex computational tasks. The MCU may be the central unit configured to run embedded software/firmware and responsible for control and coordination data exchange between circuits and electronic components.

In another embodiment, the MCU may be in connection with a dedicated digital signal processing (DSP) unit for advanced analog and/or digital signal processing.

In some embodiments, the MCU and/or DSP may be configured to operate at least one artificial neural network. An artificial neural network may be trained and configured to execute algorithms such as one or more of: speech recognition, voice-to-text, image classification, enhanced situational awareness, 3D directional sound processing, advanced signal processing, active noise cancellation, earcup cavity volume detection etc.

FIG. 3 schematically illustrates a block diagram example of the communication module 106 of the communication system 102 according to one or more embodiments of the disclosure. The digital signal processing unit (DSP) 304 may be a dedicated processor for handling audio mixing and processing tasks to ensure correct audio distribution across, for example speaker units (e.g. 212a, 212b) and microphones (e.g. 234a, 234b,236a, 236b, 214) of a hearing protection headset 104 (see FIG. 2A-FIG. 2D). The MCU 302 and DSP 304 units may be separate processor chips or operate in conjunction in a single chip set. In the present example in FIG. 3, the processors (e.g. 302, 304) may be in connection with a memory 306 component for storing code, setting and instructions such as a flash memory and a random-access memory (RAM). The MCU 302 may further be in connection with additional electronic components such as a USB PHY 308 hardware component that may act as an interface between the digital signals of a USB controller 310 and signals on an USB bus used for administrating and negotiation of digital data communication protocols with connected peripheral communication devices 110 with digital capabilities. A Universal Serial Bus (USB) communication scheme is "host controlled" meaning that there can only be one USB host per bus (i.e. physical connections and communication pathways that enable data transfer between various components).

The USB controller 310 may, in one embodiment, comprise a Host Negotiation Protocol (e.g. such as the On-The-Go specification) which allows two devices (e.g. the communication module 106 and the peripheral communication device 110) to negotiate the roles of "USB host" and "USB peripheral" (i.e. counterpart of USB host). The USB host may be responsible for undertaking all transactions and scheduling bandwidth. Data can be sent by various transaction methods using a token-based protocol as generally known in the art. In an advantageous embodiment, the communication system 102 may be configured as a USB peripheral, preferably a USB audio peripheral (e.g. a USB headset) when connected to a peripheral communication device 110 acting as a USB host, such as a smartphone, Tablet or personal computer, as the communication module 106 may utilize the set of standard drivers and software components typically comprised on a smartphone (e.g. EUD), Tablet or personal computer thereby not requiring any pre-configuration of a USB host type peripheral communication device 110 to enable data exchange (e.g. transmit one or more control-instructions).

In some embodiments, the communication module 106 may contain a power manager 312. The power manager 312 may be configured for controlling power routing (e.g. via the electrical receptacle interface 218), components and an internal power source 314 (e.g. in the battery compartments 226). The power manager 312 may additionally be configured to manages power operations of the communication system 102 such as for powering the communication system 102 itself, draw power from a connected peripheral communication device 110 with power sharing capabilities and/or directing power to charge a connected radio or EUD. The power manager 312 may additionally be configured to register if the communication system 102 is connected to an external power source, such as via a vehicle like an armored vehicle 124, helicopter or boat, and in response power the communication system 102 directly from the external power source.

In one embodiment, the communication module 106 may comprise one or more wireless module(s). According to the present example in FIG. 3, such wireless module(s) may be a Bluetooth or near-field wireless communication component 318 in connection with a Wi-Fi / Bluetooth antenna 326 for short range wireless communication with additional devices used by the user 100 for providing a wPAN (wireless personal area network). Additionally, or alternatively the communication module 106 may in one embodiment comprise a Digital Enhanced Cordless Telecommunications (DECT) module 316. The DECT module 316 may be in connected to a dedicated DECT antenna 328 arrangement configured to operate in both a star topology and a mesh topology together with other users (e.g. Each individual other user having a similar configuration as the user 100 displayed in FIG. 1B) via their equivalent communication systems.

In one embodiment, the communication module 106 may contain a user interface (UI) handler 320. The UI handler 320 may be configured for communication with a user interface 108 to provide user instructions to the communication module 106 by for example pushing a tangible control, such as a push button (e.g. see 224a-224c) and/or a toggle switch 246.

For example, a push-to-talk actuator module 324 may be signaled by the UI handler 320 to transmit a control-signals associated with push-to-talk (PTT) control-instruction to a transceiver module of a connected radio 110 to establish a person-to-person voice communication by transmitting a voice message using RF. Thus, when a user 100 activates the one or more of the tangible controls (e.g. 224a-224c, 246) on the user interface 108 a Push-To-Talk (PTT), a Carrier Operated Relay (COR) and/or a Carrier Operated Switch (COS) signal (control-instruction) may be generated or triggered by the actuator module 324 and transmitted to the connected peripheral communication device 110. The control-instruction may operate by generating a control-signal that switches between logic levels (typically +5V and Ground) in the transceiver module, indicating the activation of the transmitter by activating a squelch circuit or similar in the peripheral communication device 110 thereby allowing the connected peripheral communication device 110 to transmit a audio signa, preferably from the user when speaking via the boom microphone 214.

In one embodiment, a CODEC module CODEC 322 may additionally form part of the communication module 106.

The CODEC module 322 may be used for communication with an analog peripheral communication device 110 for transforming analog audio signals into a digital format (ADC) and vice versa (e.g. digital-to-analog (DAC) conversion) within a single unit. An analog audio signal may be a continuous electrical representation of sound waves. The analog audio signal may thus directly mirror the fluctuations in air pressure caused by sound, varying in voltage or current to correspond with the original acoustic signal.

In one embodiment, the communication module comprise active noise reduction (ANR) circuit 330 configured to attenuate the external sounds from the harsh environment by generating anti-noise signals via said speaker units (e.g. See 212a, 212b in FIG. 2A-FIG. 2D) that destructively interfere with the external sound. In some embodiments, the ANR circuit 330 may form part of the MCU 302 and/or DSP 304 units. Alternatively, the ANR circuit 330 may be a dedicated processing circuits separate to the MCU 302 and/or DSP 304.

In one embodiment, the communication module 106 may comprise a system on a chip (SoC) coupled with additional electronic circuits and devices. The SoC may be a Qualcomm QCC5181 or a Snap Dragon S7 sound chipset constraining one or more microprocessors, such as an MCU 302, and a DSP 304. The processing circuits of the system on a chip (SoC) may be configured to run embedded software and dedicated firmware such as programs or control functions. Examples of such functions may be:
A "hear through" function, enabling the communication system 102 to provide artificial hearing. By artificial hearing, external sounds that the user 100 might want to hear are detected and captured (e.g. via the ambient microphones 236a, 236b) whereas the sound signals may be processed in real-time by the DSP 304 before the external sounds are reproduced by speakers (e.g. 212a, 212b) inside the headset 104. This function providing the effect of making the headset "transparent" in terms of listening to the surroundings. Thus, the hear-through function in the headset 104 may operates by capturing external sounds using left ambient microphone 236a and right ambient microphone 236b, subsequently processing the corresponding audio signal of the captured external sounds from the left 236a and right ambient microphone 236b individually, and reproducing the captured external sounds through the corresponding left speaker unit 212a and right speaker unit 212b, thus the external sounds captured by the left ambient microphone 236a may be directed to the left speaker unit 212a and the external sounds captured by the right ambient microphone 236b may be directed to the right speaker unit 212b. This artificial hearing allows the user 100 to perceive ambient sounds while wearing the hearing protection headset 104. The processor may apply filtering, gain adjustment, or noise suppression to enhance for example speech clarity and reduce unwanted noise. Additionally, the processor may apply a head-related transfer function (HTF), e.g. Specifically designed for the user or a generic type of HRTF to enhance the directional determination of the external sounds when experienced by the user via the artificial hearing function. Hence, a HRTF may model how the head and ears of the user may shape incoming sound, enabling spatial perception through interaural time differences (ITD), interaural level differences (ILD), and spectral cues. When using artificial hearing, HRTF-based processing may apply binaural cues to synthesize directionality of the external sounds. This ensures that the reproduced external sounds in the headset's speakers (e.g. 212a, 212b) mimic natural hearing, maintaining spatial awareness by preserving the perceived location of sounds. By adjusting timing, intensity, and frequency response, the artificial hearing allows users 100 to hear their surroundings naturally while wearing the hearing protection headset 104. Real-time processing may be achieved by using a dedicated artificial hearing processing circuitry as a separate circuit or as a part of other processors such as the DSP 304 or MCU 302.

An "Active Hearing Protection" function, for providing active haring protection. This function co-function with the "hear through" function in which ambient sound recorded by the ambient microphones 236a, 236b are allowed to be transmitted to the user 100, where the "Active Hearing Protection" function may limit the overall amplitude of the sound signals emitted by the speaker units 212a, 212b so as to protect the user's 100 hearing. This feature provides artificial hearing and situational awareness by enabling the user 100 to hear and react to ambient external sounds, while protecting the user's hearing from overly loud sounds such as heavy machinery or a gunshot etc. which is highly advantageous when used in the dismounted configuration 114 in harsh and demanding environments, as it preserves the users 100 ability to utilize the hearing sense. The "Active Hearing Protection" function may advantageously function with the sound signal received (Rx signals) via a connected peripheral communication device 110, such that audio played to the user 100 from a remote destination may also be limited in overall amplitude to protecting the user's hearing if for example another user may fire a weapon with voice-activated-exchange (VOX) activated, causing the second user to transmit the sound of the gunshot to the first user which otherwise (e.g. without the active hearing protection function) could cause the speaker units 212a, 212b to emit sound in a dangerous volume for the hearing ability of the user 100.

A "Active Noise Reduction (ANR)" function for performing automatic noise reduction (ANR) inside the left 200a and right earcup 200b cavities of the hearing protection headset 104 via dedicated circuits to perform "feed-forward noise reduction" using the ambient microphones (e.g. 236a and 236b) and the speaker units (e.g. 212a, 212b) and/or "feed-backwards noise reduction" using the monitor microphones 234a, 234b and the speaker units 212a, 212b. The ANR function may use one or more dedicated electronic ANR circuits 330 (e.g. Forming part of the MCU 302, DSP 304 and/or a separate electronic circuit) to generate anti-noise signals (e.g. via the speaker units 212a, 212b) that destructively interfere with ambient sound to reduce it thereby providing an improved hearing protections relative to only utilizing passive sound attenuation. The ANR function may be configured to operate in at least a first ANR mode or a second ANR mode or even a third ANR mode. The first ANR mode is applied when the volume inside each of the earcups 200a, 200b corresponds to the first cavity volume when the hearing protection headset 104 is in the dismounted mode (e.g. adapter elements not mounted, see dismounted configuration 114 in FIG. 1A). The second ANR mode is applied then the volume inside each of the earcups 200a, 200b corresponds to the second cavity volume, when the hearing protection headset 104 is in the mounted mode (e.g. Adapter elements mounted, see mounted configuration 116 in FIG. 1B). More details on the ANR are provided in the following.

### Active noise reduction (ANR)

In one embodiment, the left earcup and the right earcup each comprise an internal microphone configured to receive sound from inside the earcups when worn by a user, and a processor configured to provide the ANR by continuously monitoring the sound from inside the earcups and generating an inverse sound signal that is in counter phase with the detected internal sound. It is here noted that the detected internal sound may depend on the cavity volume, and this embodiment may provide a setting, where the ANR is dependent on the cavity volume.

One purpose of providing ANR in the headset, in addition to the passive noise reduction, is to improve the noise reduction by supplementing the passive noise attenuation. Accordingly, the ANR, as here described, may increase hearing the protection and provide additional comfort in noisy scenarios.

There are two approaches for implementing the ANR functionality - a feedforward approach and feedback ANR approach (or a hybrid by combining the two). The main difference between a feedforward ANR approach and a feedback ANR approach is the placement of microphones relative to the loudspeaker (e.g. 212a and 212b) and subsequent control method. Feedback ANR utilizes a microphone placed inside the earcup (e.g. 234a 234b), close to the loudspeaker (e.g. 212a, 212b), and records any noise which enters the earcup (e.g. 200a, 200b) when worn by the user. This method is best suited to reduce static low frequency noise due to the processing delay of measuring at the point where noise reduction is desired. Feedforward ANR utilizes a microphone placed outside the earcup (e.g. 236a, 236b) and as such measures the external noise before it enters the earcup and arrives at the speaker (212a, 212b). This approach is better at high frequency noise reduction, but less precise at it does not monitor the actual noise in the earcup.

Accordingly, in one embodiment, the headset comprises the internal microphone (e.g. left Monitor microphone 234a and right monitor microphone 234b) to provide feedback ANR. Both the mounted and dismounted mode (e.g. see. 116 and 114 in FIG. 1A and FIG. 1B) may benefit from having an ANR circuit 330 configured to perform feedback ANR since the user in both modes is likely to be exposed to a high level of external noise from the harsh and demanding environment. The user in the mounted mode (e.g. Mounted configuration 116 in FIG. 1B) may particularly be exposed to engine and vehicle noise over long time periods. Feedback ANR may be advantageous over feedforward ANR, because in extremely noisy conditions, acoustic overload of the microphone could become a problem for feedforward ANR due to microphone overload. Since the feedback ANR has the microphone inside the earcup, the microphone is not as exposed and may therefore not experience the acoustic overload.

The ANR functionality may be tuned by modelling the transmission of audio (transfer function) between speaker (e.g. 212a, 212b) and in-cup microphone (e.g. 234a, 234b) for feedback ANR or from outer microphones (e.g. 236a, 236b) through the earcup and to the ear drum for feedforward ANR. The transfer functions are dependent on the volume/earcup design (e.g. A first cavity volume or second cavity volume), characteristics of the microphone and speaker and transducer placements.

Based on the model, which can be extracted from measurements, a ANR filter may be tuned to fit the transfer function in a desired frequency region. A filter may in essence be an equalizer which shapes the sounds measured by the microphone (e.g. 234a, 234b, 236a, 236b) before playing it back on the speakers as anti-noise. When designing/tuning a feedback ANR filter it may be designed such that the filter does not introduce excessive phase shifting or feedback oscillation as this can reduce the performance or worst case create howling.

In addition to the ANR filter, a compensation filter may also be applied to the incoming (Rx) signal (e.g. Audio signal received from a first type of peripheral communication device), like echo cancellation, as it is not desired to reduce the sounds coming from communication equipment or a situational awareness signal (e.g. Artificial hearing).

Accordingly, in a preferred embodiment, the ANR functionality comprises a ANR filter, preferably a tunable filter and/or a compensation filter.

In one embodiment the first ANR mode comprise a first ANR filter, preferably a tunable filter and/or a compensation filter and the second ANR mode comprise a second ANR filter preferably a tunable filter and/or a compensation filter.

In some embodiments, the tunable filter is tuned/adapted according to the cavity volume. Such that the first ANR filter may be tuned according to the first cavity volume and the second filter may be tuned according to the second cavity volume.

In one embodiment, the headset is configured to operate in at least a first ANR mode or a second ANR mode, wherein the first ANR mode comprise a tunable filter being tuned/adapted according to the first cavity volume and wherein the first second ANR mode comprise a tunable filter being tuned/adapted according to the first cavity volume.

In one embodiment, and when in the first ANR mode, the ANR circuit is configured to operate at a first level of noise reduction that is dependent on the first cavity volume (e.g. See first volume 254 in FIG. 2D), preferably such that the first level of noise reduction is matched to the first cavity volume (e.g. See first volume 254 in FIG. 2D) and its corresponding passive sound attenuation. Further, in this embodiment, and when in the second ANR mode, the ANR circuit is configured to operate at a second level of noise reduction that is dependent on the second cavity volume (e.g. See first volume 254 combined with void volume 256 in FIG. 2D), preferably such that the second level of noise reduction that is matched to the second cavity volume (e.g. See first volume 254 combined with void volume 256 in FIG. 2D) and its corresponding passive sound attenuation.

As previously described, this may provide a communication system for communicating in a harsh environment that is configured to adapt to a first situation with low noise and to a second situation with high noise.

This way, the headset may apply a ANR function optimized for the specific earcup cavity volume in both a dismounted configuration 114 and a mounted configuration 116. The first ANR mode may be tuned such that the transfer function applied corresponds with the first cavity volume and the second ANR mode may be tuned, such that the transfer function applied corresponds with the second cavity volume. In another scenario, the first ANR mode may apply a filter optimized to attenuate external sounds in a first range of frequency bands, such as following a pink noise distribution profile. The second ANR mode may apply a filter optimized to attenuate external sounds in a second range or frequency bands, such as in the low frequency spectrum 20-1000 Hz, corresponding to the noise profile within an armored vehicle. This way the ANR circuit may apply a first ANR mode or a second ANR mode adapted to the specific noise profile and loudness experienced by a user operating in a dismounted configuration or a mounted configuration (e.g. See FIG. 1A, FIG. 1B)

In a related embodiment, the first level of noise reduction is lower than the second level of noise reduction, such that when in the first ANR mode, external sounds are reduced by the ANR circuit less than when in the second ANR mode.

The first level of noise reduction and the second level of noise reduction may be measured in terms of overall average decrees in sound-pressure-level (SPL) measured in decibel (dB) as an average covering a wide frequency range. Alternatively, the first level of noise reduction and the second level of noise reduction may be measured in terms of the decrease in sound-pressure-level (SPL) measured in decibel (dB) in a narrow frequency range. Or the first level of noise reduction and the second level of noise reduction may be measured using the Noise Reduction Eating (NRR) standard method.

In another embodiment, and when in the first ANR mode, artificial hearing is enabled, and when in the second ANR mode, artificial hearing is disabled. This may adapt situational awareness to different situations depending on which ANR mode the headset is set to operate in.

In some embodiments, the first ANR mode is the same as the second ANR mode. This may for example be the case if the passive noise reduction is sufficient to reduce the noise dependent on the environment.

However, in most embodiments, the first ANR mode differs from the second ANR mode, preferably wherein the first ANR mode and the second ANR are different as described in various embodiments above.

In a preferred embodiment, the left earcup 200a and the right earcup 200b each comprise an ambient microphone (e.g. A left ambient microphone 236a and a right ambient microphone 236b) configured to receive the external sounds, and a processor (e.g. 302, 304) configured to provide the artificial hearing by transmitting the received external sounds to the user 100 via the left and the right earcup, thereby providing the user with the ability to hear the surroundings when wearing the headset.

### Adapter element detection method

In most embodiments, said headset is further configured to instruct the ANR circuit to operate in the first ANR mode or in the second ANR mode. For example, said headset may comprise a processor configured to process output from the one or more sensors (or from the internal microphone) and to transmit operation instructions to the ANR circuit.

In another embodiment, said headset configured to automatically detect that the headset i worn by a user and in response thereof set the ANR circuit to operate in the first ANR mode or the second ANR mode.

Advantageously, the headset is capable of detecting that it is worn by a user, such as placed to cover ears of the user and forming an acoustic barrier to protect the hearing of the user from external sounds. This way, the headset may be configured to adapt the active noise cancellation mode applied between a first ANR mode and a second ANR mode. Preferably, the headset is capable of detecting if the headset is worn or not, and if worn, the headset may automatically detect if the earcup cavity volume corresponds to a first cavity volume or a second cavity volume and adjust the ANR mode accordantly.

In some embodiments, the headset is configured to automatically detect whether the ANR circuit is set to operate in the first ANR mode or the second ANR mode. As described above, this may be based on monitoring the sound from inside the earcups.

However, in other embodiments, the headset comprises one or more sensors located in and/or on said earcups to detect whether the adaptor element is removably placed between the opening and the flexible seal or not, thereby enabling said headset to automatically detect whether the ANR circuit is set to operate in the first ANR mode or the second ANR mode.

In related embodiments, the one or more sensors is an optical sensor, and/or an electronic sensor, and/or an imaging sensor, and/or an audio sensor, and/or vibration sensor, preferably where the audio and/or vibration sensor is configured to record audio or vibration, respectively, from the earcup, such as from a signal generator in the earcup, thereby detecting whether the recorded audio or vibration matches that to the first cavity volume or the second cavity volume.

An automatic detection may be advantageous as this may reduce the stress of the user, such as a military person using the headset, for example in a combat zone, where stress may be very high.

In one example the headset may comprise a contrast detection sensor located within the cavity of the primary housings of each earcups (e.g. 228a, 228b) having an active sensing area in the direction towards the opening of the said earcups. The contrast sensor may be a photoelectronic sensor configured to detect presence or absence of light. When the headset is powered on, the contrast sensor may continuously perform readings every 0.1-5 second related to the presence or absence of light. This way, the headset is capable of detecting when the headset is worn by the user, as the flexible seal of each earcup not only provides an acoustic seal as previously explained but additionally blocks out any ambient light from the outside. In case the absence of light threshold is measured during a time interval of consecutive readings, the headset may conclude that the headset is worn by the user.

When the headset has confirmed that it is worn by a user, the headset may instruct the ANR circuit to apply either the first ANR mode or the second ANR mode. It is advantageous that the ANR circuits initiate either the first ANR mode or the second ANR mode subsequent to confirming that the headset is worn by a user, such that the sound level in the earcup cavities are stable (no leaks) and the microphones (234a, 234b) and speakers (212a, 212b) levels are equilibrated to avoid unwanted feedback loops and achieve optimal ANR performance.

In one embodiment, the loudspeakers and internal microphones in each may be used for detecting if the hearing protection headset is worn by a user or not and detect if the earcup cavity volume is a first cavity volume or a second cavity volume.

The headset 104 may be configured to generate a high frequency sound wave pulse, such as in the near ultrasonic range (e.g. 15-20kHz) via the speaker units (e.g. 212a,212b), and measure the time-of-flight (TOF) until the echo from the sound wave pulse reaches the internal microphone (e.g. 234a, 234b). The TOF may be used firstly to detect that the headset 104 is worn by a user 100 and that the earcup cavity volume corresponds to a first cavity volume or a second cavity volume. The sound pulse generated by the speaker unit (e.g. 212a,212b) would travel the length (e.g. Depth of the first cavity volume or second cavity volume), reach the ear and skin of the user and bounce back towards the microphone (e.g. 234a,234b). In case the headset is not worn by a user, no echo would be registered by the microphone within a short time interval. In case the headset 104 is worn, the first cavity volume or second cavity volume may be detected based on a change in the TOF measured. For example, with reference to the illustration in FIG. 2D, if the right rim 250b has a thicknessed 10 mm (e.g. Depth of the void volume 256), the presence of the right adapter ring 248b would result in a increased TOF of about 60 ms compared to the situation without the right adapter ring 248b. In case the headset 104 may be operating the microphones (e.g. 234a,234b) at a 25 kHz clock sampling frequency, the presence of the right adapter ring 248b would be detectable by about 3 microphone sample intervals, being sufficient to confidently determine the increase in TOF.

The same TOF detection method (e.g. Emit sound pulse from a speaker and measure the TOF of the echo by an internal microphone) may simultaneously or individually be performed by the hearing protection headset 104 in both the right earcup 200b and the left earcup 200a (e.g. using the left speaker unit 212a and left monitor microphone 234a.). It is highly advantageous to use sound waves in the near-ultrasonic range, as those frequencies are not detected by the human ear and brain by taking into account gradual loss of sensitivity to higher frequencies with age (e.g. which typically applies to users operating in harsh and demanding environments), thus, the headset 104 may perform the TOF detection method while being unnoticed by the user. This provides the additional advantage that the TOF detection method may be performed by the headset with regular intervals during usage, such that changes to the earcup cavity volume or wearing style may be detected almost instantaneously automatically.

In another example, an artificial neural network may be trained and configured to perform earcup cavity volume detection, such as detecting a first cavity volume or a second cavity volume based on image classification. A miniature image sensor med be mounted in each of the left earcup 200a and right earcup 200b, preferably in combination with a small light source. The image sensor may be configured to acquire image data of the interior of each earcup, and the trained neural network may be trained to classify the images to detect if the headset is worn by the user 100 or not and if an adaptor element is present or not. Invidiously, the LED light source is an IR light source to avoid any flashing of visible light from the headset and the image sensor is an IR sensitive sensor.

### User interface

In one embodiment, said headset comprises a user interface configured to manually select whether the ANR circuit is set to operate in the first ANR mode or the second ANR mode. Such a configuration may provide a simple and low-cost headset.

In some embodiments, said user interface is fixed on said earcup.

In a preferred embodiment, said user interface is removably mounted on said earcup via a user interface connector.

In a most preferred embodiment, said headset comprises a user interface connector configured to removably connect a user interface, and wherein said headset is configured such that when the user interface is not removably connected to the user interface connector, then the ANR circuit is set to operate in the first ANR mode, and when the user interface is removably connected to the user interface connector, then the ANR circuit is set to operate in the second ANR mode.

In one embodiment, the user interface comprises one or more tangible controls that are manually operably by the user. In another embodiment one of said tangible controls is a switch. The switch may for example allow the communication module to transmit the one or more control-instructions to the at least first type of peripheral communication device. For example, in some embodiments, the one or more control-instructions is/are in the form of Push-to-Talk (PTT) signals and wherein the at least first type of peripheral communication device in the form of a radio. However, other intercom systems may act as a communication hub, bridging communication between connected users (e.g. crew members) and connected communication devices, and a radio is just an example. Nevertheless, regardless of the intercom system, the tangible user interface may enable crewmembers to communicate both internally and externally, thus allows for coordination of tasks, sharing of information, and quick decision-making.

By having a user interface connector configured to removably mount the user interface, such as described above, the user may enable that in the second ANR mode, the user is able to transmit the one or more control-instruction to the at least first type of peripheral communication device, such as a radio. As radio communication is typical inside a vehicle, the second ANR mode is optimal for a situation where the user is inside the vehicle. The user may learn that when inside the vehicle, it may be a procedure to plug in the user interface in the headset, whereby the second ANR mode is activated. In this manner, the user need not to consider whether the headset is to be set in the first ANR mode or the second ANR mode and the device does it therefore based on the procedural action from the user. The above-described embodiment thus provides efficient manual selection of noise suppression.

In one example, the user interface connector may be the left connector interface 216a and/or right connector interface 216b as displayed in FIG. 2A-FIG. 2D, and the

FIG. 4 schematically illustrates an example of the communication system comprising a left connector interface 216a and a right connector interface 216b in connection with a communication module 106 according to the disclosure. The communication module 106 (e.g. See FIG. 3) may be in contact with the left connector interface 216a comprised by the left earcup 200a and right connector interface 216b comprised by the right earcup 200b of the hearing protection headset 104 (e.g. See FIG. 2A-FIG. 2D). Both the left connector interface 216a and the right connector interface 216b may be configured to releasably mount and connect a first type of accessory device and/or a second type of accessory device. In the example in FIG. 4 the left connector interface 216a is about to releasably mount and connect the boom microphone 214 (e.g. A first type of accessory device) whereas the right connector interface 216b is about to mount and connect a toggle switch 246 (a second type of accessory device). However, it should be noted that the boom microphone 214 could also be connected to the right connector interface 216b and similar, the toggle switch 246 could also connect to the left connector interface 216a. Upon connection, the toggle switch 246 might be recognized by the communication module 106 which then instruct the ANR circuit to apply the second ANR mode. When the toggle switch 246 is not removably mounted to the right connector interface 216b (e.g. or the left connector interface 216a), then the ANR circuit 330 is set to operate in the first ANR mode (e.g. the default mode). This way, the connection of the toggle switch 246 may be used as a proxy detecting mechanism for instructing the ANR circuit to apply the second ANR mode associated with the earcup cavity volume corresponding to the second cavity volume. The toggle switch 246 may additionally provide an additional or alternative user interface for the communication system 102 relative to the user interface 108 as displayed in in relation to FIG. 2A and elsewhere. The toggle switch 246 may comprises one tangible control that is manually operably by the user 100, in the form of a joystick portion 402. The tangible control element (e.g. Joystick portion 402) may support at least one operation state. The toggle switches 246 may be a 3-position switch allowing for three operation states. The user 100 may manually operate the toggle switch 246 by positioning the joystick portion 402 in different orientations, such as a first operation state may correspond to a first position 404a, a second operation state may correspond to a second position 404b, and a third operation state a third position 404c. The operation states of the toggle switch 246 may generate one or more control-signals and control-instructions by the communication module 106 to a connected peripheral communication device 110. For example, the first position 404a may disable the boom microphone 214, such that no voice signal of the user 100 may be transmitted via any connected peripheral communication devices 110 or communication hub 118 (e.g. And Interrupt any Push-to-Talk (PTT) signals), the second position 404b may generate a Push-to-Talk (PTT) signal to a first net or first talk group of a connected peripheral communication device 110 or communication hub 118 and enable VOX such that a voice signal may be transmitted via the first net or talk group of a peripheral communication device 110 or communication hub 118 when a speech signal of the user 100 is detected. The third position 404c may be spring-loaded meaning that the position returning to the previous position (e.g. Second position 404b) if not activated constantly by the user 100. The third position 404c may generate a Push-to-Talk (PTT) signal to a second net or second talk group of a connected peripheral communication device 110 or communication hub 118 when activated and allow audio data from the boom microphone 214 to be transmitted to second net or second talk group of the peripheral communication device 110 or communication hub 118.

The functionality of the toggle switch 246 may be particularly relevant when the communication system 102 in worn by a user 100 being a combat vehicle crewman (CVC) operating in a military armored vehicle 124 (e.g. In the mounted configuration 116 in FIG. 1B). In such as scenario, the CVC 100 may be connected to a vehicle mounted intercom system (e.g. Communication hub 118 in FIG. 1B) along with other crew members (e.g. Also waring respective communication systems according to the disclosure) and one or more radios for communication outside of the vehicle. In such a configuration, the toggle switch 246 may enable a CVC 100 to communication with crew members via the intercom system (e.g. Communication hub 118 in FIG. 1B) when the joystick portion 402 is in the second position 404b (e.g. Fia a first talk group of the communication hub 118) and communicate, via the vehicular integrated radio 110, with externally people when the joystick portion 402 is in the third position 404c thereby providing clear and undistorted communication for CVC 100 operating in harsh and demanding environments. The tangible design of the toggle switch 246 having a joystick portion 402 is advantageous for the CVC as the manual operation of the user interface (e.g. Toggle switch 246) should be easy and intuitive to use even with thick gloves where well-defined movements are appreciated for unambiguous control on the communication.

Some preferred embodiments have been shown in the foregoing, but it should be stressed that the invention is not limited to these but may be embodied in other ways within the subject matter defined in the following claims.

It should be emphasized that the term "comprises/comprising" when used in this specification is taken to specify the presence of stated features, elements, steps or components but does not preclude the presence or addition of one or more other features, elements, steps, components or groups thereof.

In the claims enumerating several features, some or all of these features may be embodied by one and the same element, component or item. The mere fact that certain measures are recited in mutually different dependent claims or described in different embodiments does not indicate that a combination of these measures cannot be used to advantage.

In the claims, any reference signs placed between parentheses shall not be constructed as limiting the claim. The word "comprising" does not exclude the presence of elements or steps other than those listed in a claim. The word "a" or "an" preceding an element does not exclude the presence of a plurality of such elements.

The mere fact that certain measures are recited in mutually different dependent claims does not indicate that a combination of these measures cannot be used to an advantage.

It will be apparent to a person skilled in the art that the various embodiments of the invention as disclosed and/or elements thereof can be combined without departing from the scope of the invention as defined in the claims.

## Claims

1. A communication system (102) for communicating in a harsh environment, comprising:
a hearing protection headset (104) to be worn by a user (100) in the harsh environment, said headset comprising:
- a left (200a) and a right earcup (200b),
wherein each of said earcup comprises:
- a speaker unit (212a, 212b);
- a cavity with an opening adapted to accommodate the ear of the user (100);
- a flexible seal (204a, 204b) around the periphery of the opening to form an ear opening, wherein the flexible seal (204a, 204b) is configured to acoustically seal the cavity when worn by the user; wherein each of said earcup (200a,200b) and flexible seal defines a (210), wherein the first cavity volume contributes to provide passive sound attenuation, whereby each earcup is configured to attenuate external sounds from the harsh environment,
an adaptor element having a central void and a rim (250a, 250b) having a thickness such that the central void defines a predetermined void volume (256), and wherein the adaptor element (120,248a,248b) is configured to be removably arranged between the opening and the flexible seal (204a,204b) such that the rim (250a, 250b) rest against the periphery of the opening, thereby effectively increasing the first cavity volume (210, 254) to a second cavity volume defined by the first cavity volume (210,254) and the void volume (256), thereby increasing the passive sound attenuation,
wherein said headset (104) further comprise an active noise reduction (ANR) circuit (330) configured to attenuate the external sounds from the harsh environment by generating anti-noise signals via said speaker units (212a,212b) that destructively interfere with the external sounds, wherein the ANR circuit (330)) is configured to operate in two ANR modes:
- a first ANR mode applied when the adaptor element is not removably placed between the opening and the flexible seal, or
- a second ANR mode applied when the adaptor element is removably placed between the opening and the flexible seal.

2. The communication system (102) according to claim 1, wherein said headset (104) is configured to automatically detect whether the adaptor element is removably placed between the opening and the flexible seal or not, thereby enabling said headset to automatically detect whether the ANR circuit is set to operate in the first ANR mode or the second ANR mode.

3. The communication system according to claim 2, wherein said headset comprises one or more sensors located in and/or on said earcups to detect whether the adaptor element (120,248a,248b) is removably placed between the opening and the flexible seal (204a, 204b) or not, thereby enabling said headset (104)) to automatically detect whether the ANR circuit (330) is set to operate in the first ANR mode or the second ANR mode.

4. The communication system according to claim 3, wherein the one or more sensors is an optical sensor, and/or an electronic sensor, and/or an imaging sensor, and/or an audio sensor, and/or vibration sensor, preferably where the audio and/or vibration sensor is configured to capture audio or vibration, respectively, from the earcup, such as from a signal generator in the earcup (200a, 200b), thereby detecting whether the captured audio or vibration matches with the first cavity volume (210,254) or the second cavity volume.

5. The communication system (102) according to any one of claims 1 to 4, wherein said headset is further configured to instruct the ANR circuit to operate in the first ANR mode or in the second ANR mode.

6. The communication system according to claim 5, wherein said headset comprises a processor (302,304) configured to process output from the one or more sensors and to transmit operation instructions to the ANR circuit (330) instructing the ANR circuit (330) to operate either in the first ANR mode or in the second ANR mode.

7. The communication system (102) according to any of the preceding claims, wherein said headset further comprises a communication module (106) configured to:
- connect with at least a first type of peripheral communication device (110),
- receive at least one audio signal from at least the first type of peripheral communication device (110), and
- transmit the at least one audio signal to the speaker unit (212a) in the left earcup (200a) and/or a speaker unit (212b) in the right earcup (200b).

8. The communication system (102) according to claim 7 as dependent on claim 6, wherein the processor (302,304) is part of the communication module (106).

9. The communication system (102) according to any of the preceding claims, said communication module is further configured to:
transmit one or more control-instruction(s) to the first type of peripheral communication device and wherein
wherein said headset (104) comprises a user interface (108,246) configured to manually select whether the ANR circuit (330) is set to operate in the first ANR mode or the second ANR mode.

10. The communication system (102) according to any of the preceding claims, wherein when in the first ANR mode, the ANR circuit (330) is configured to operate at a first level of noise reduction that is dependent on the first cavity volume (210,254), preferably such that the first level of noise reduction is matched to the first cavity volume (210,254) and its corresponding passive sound attenuation, and wherein when in the second ANR mode, the ANR circuit (330) is configured to operate at a second level of noise reduction that is dependent on the second cavity volume, preferably such that the second level of noise reduction that is matched to the second cavity volume and its corresponding passive sound attenuation.

11. The communication system (102) according to claim 10, wherein the first level of noise reduction is lower than the second level of noise reduction, such that when in the first ANR mode, external sounds are reduced by the ANR circuit (330) less than when in the second ANR mode.

12. The communication system (102) according to any of the preceding claims, wherein when in the first ANR mode, an artificial hearing is enabled, and when in the second ANR mode, the artificial hearing is disabled.

13. The communication system (102) according to claim 12, wherein the left earcup (200a) and the right earcup (200b) each comprise an ambient microphone (236a, 236b) configured to capture the external sounds, and wherein the communication system (102) further comprises a processor (302,304) configured to process the captured external sound signals and provide the artificial hearing by transmitting the processed captured external sound signals to the user (100) via the corresponding left (200a) and the right earcup (200b), thereby providing the user (100) with the ability to hear the surroundings when wearing the headset (104).

14. The communication system (102) according to any of the preceding claims, wherein the left earcup (200a) and the right earcup (200b) each comprises an internal microphone (234a,234b) configured to capture internal sound from inside the respective earcup (200a,200b) when worn by a user (100), and wherein the communication system (102) further comprise a processor (302,304) configured to provide the ANR functionality by, e.g. or preferably continuously, monitoring the respective captured internal sound and generating a respective inverse sound signal that is in counter phase with the respective captured internal sound.

15. A head-worn system comprising the communication system (102) according to any one of claims 1 to 14.
